# EUROPÄISCHE PATENTANMELDUNG

(11) **EP 0 628 810 A2**
(43) Veröffentlichungstag der Anmeldung: **14.12.1994**
(21) Anmeldenummer: 94250126.3
(22) Anmeldetag: 16.05.1994
(51) Int. Cl.: G01N 27/416, G01N 33/00

(54) **Elektrochemisches Gasspurenmesssystem mit Funktionskontrolle**

(30) Priorität: 07.06.1993 DE 4318891
(71) Anmelder: Compur Monitors Sensor Technology GmbH, D-81539 München (DE)
(72) Erfinder: Braden, Christoph, Dr., 50937 Köln (DE); Pross, Wilhelm, 93449 Geigant (DE); Pfenning, Dirk, Dr., 51381 Leverkusen (DE); Rietzl, Kurt, 86899 Landsberg/Lech (DE)
(74) Vertreter: Wagner, Karl H., Dipl.-Ing.

(57) **Zusammenfassung**

Das Gasspurenmeßsystem besteht aus einem elektrochemischen Sensor (1) mit mindestens einer Arbeitselektrode, einer Gegenelektrode und gegebenenfalls einer Referenzelektrode. Das vom Sensor erzeugte, mit der Gaskonzentration korrelierte Meßsignal wird mit Hilfe einer analogen Auswerteelektronik (2) verstärkt und angezeigt. Die Auswerteelektronik (2) ist mit einer Diagnoseschaltung (4, 5, 6) verbunden, die während eines Testzyklus bei einem Zweielektrodensensor, das zwischen Arbeitselektrode und Gegenelektrode und bei einem Dreielektrodensensor, das zwischen Arbeitselektrode und Referenzelektrode eingestellte Potential nach einer vorgegebenen Zeitfunktion vorübergehend verändert, was eine Änderung des Stromsignals an der Arbeitselektrode hervorruft. Diese Stromsignaländerung oder auch ein davon abgeleiteter Meßwert wird von der Auswerteelektronik als ein für die Sensorqualität repräsentatives Testsignal erfaßt und mit einem oder mehreren vorgegebenen Sollwerten verglichen.

## Beschreibung

Die Erfindung geht aus von einem Gasspurenmeßsystem, das aus einem elektrochemischen Sensor mit mindestens einer Arbeitselektrode, einer Gegenelektrode und gegebenenfalls einer Referenzelektrode besteht und eine analoge Auswerteelektronik zur Bildung von Meßwerten aufweist.

Elektrochemische Gassensoren werden für Arbeits- und Umweltschutzzwecke vielfach zur Messung von Gasspuren bis in den ppb-Bereich eingesetzt. Ein typischer Zweielektrodensensor mit einer Arbeits- und Gegenelektrode ist z.B. in DE 24 36 261 und ein typischer Dreielektrodensensor mit einer Arbeitselektrode, einer Gegenelektrode und einer Referenzelektrode in DE 21 55 935 beschrieben. Mehrfachelektrodensensoren mit mehreren Arbeitselektroden zur simultanen Messung verschiedener Gaskomponenten sind aus DE 24 35 813 und DE 41 36 779 bekannt. Ein generelles Problem solcher Sensoren ist die Zuverlässigkeit und Verfügbarkeit der Meßwerte. Eine Fehlfunktion der Meßzelle muß in jedem Fall rechtzeitig erkannt werden, um Folgeschäden für den Anwender des Gerätes auszuschließen. Aus diesen Gründen ist es üblich, die Geräte vor der Benutzung einer Funktionskontrolle zu unterziehen. Zu diesem Zweck wird der Sensor kurzzeitig mit einem Testgas beaufschlagt und das dabei erzeugte Meßsignal mit einem Sollwert verglichen. Ein derartiger Funktionstest wird in DE 26 21 677 und DE 31 29 680 beschrieben. Ferner ist ein elektrochemischer Mehrfachelektrodensensor bekannt, bei dem die Arbeitselektroden gleichzeitig der zu messenden Gaskonzentration ausgesetzt werden und bei einem zu großen Unterschied der Meßsignale an den Arbeitselektroden eine Warnanzeige erfolgt. In diesem Fall beruht also die Funktionsüberwachung auf redundanten Meßzellen bzw. Arbeitselektroden. Ein nach diesem Prinzip arbeitender elektrochemischer Gassensor wird in DE 26 58 734 beschrieben. Derartige Anordnungen sind jedoch zwangsläufig mit einem größeren apparativen und schaltungstechnischen Aufwand verbunden.

Hier setzt die Erfindung an. Es liegt die Aufgabe zugrunde, ein Gasspurenmeßsystem auf der Basis von herkömmlichen elektrochemischen Zwei- oder Dreielektrodensensoren (in abgewandelter Form auch Multielektrodensensoren) zu entwickeln, bei dem jederzeit, d.h. auch am Einsatzort, ein einfacher elektrischer Funktionstest durchgeführt werden kann.

Diese Aufgabe wird erfindungsgemäß dadurch gelost, daß die im Gasspurenmeßsystem bereits vorhandene Auswerteelektronik zusätzlich mit einer Diagnoseschaltung versehen wird, die während eines Testzyklus das zwischen Arbeitselektrode und Gegenelektrode oder zwischen Arbeitselektrode und Referenzelektrode eingestellte Potential nach einer vorgegebenen Zeitfunktion vorübergehend verändert und die dadurch erzeugte Stromsignaländerung an der Arbeitselektrode als ein für die Sensorqualität repräsentatives Testsignal ausgibt und mit einem oder mehreren vorgegebenen Sollwerten vergleicht. Die Diagnoseschaltung ist dabei so ausgeführt, daß von dem normalen Meßmodus auf einen Testmodus umgeschaltet werden kann. Im Testmodus wird dann dem Potential an der Arbeitselektrode vorübergehend eine Testspannung überlagert und die dadurch erzeugte Stromsignaländerung (Sensorantwort) als Testsignal ausgewertet und mit bekannten Daten, die eine fehlerfrei funktionierende Sensorzelle liefert, verglichen. Auf diese Weise kann eine defekte Sensorzelle schnell erkannt werden.

Es ist auch möglich, durch eine Messung der zeitlichen Änderung des Testsignals eine Information über die Ansprechzeit des Sensors (Responsekurve) zu erhalten. In diesem Fall wird dem Potential an der Arbeitselektrode zweckmäßig ein Rechteckimpuls überlagert.

Anstelle eines direkten Vergleichs der durch das Testsignal erzeugten Stromsignaländerung an der Arbeitselektrode (Sensorantwort) kann gemäß einer bevorzugten Ausführungsform das Testsignal auch dadurch erzeugt werden, daß das Stromsignal an der Arbeitselektrode während des Testzyklus aufintegriert wird.

Gemäß einer Weiterentwicklung der Erfindung wird bei einem elektrochemischen Drei- oder Mehrelektrodensensor mit einer analogen potentiostatischen Auswerteelektronik das Potential zwischen Arbeitselektrode und Referenzelektrode während des Testzyklus nach Art eines differentiellen zyklischen Voltagramms mit einer Wechselspannung moduliert und durch Integration des in die Arbeitselektrode fließenden Stromsignals über eine Periode der Wechselspannung die von der dabei durchlaufenen Strom-Spannungshysteresekurve eingeschlossene Flache als Testsignal ausgewertet.

Vorteilhaft weist die Diagnoseschaltung einen Komparator zum Vergleich des Testsignals mit einem oder mehreren vorgegebenen, gespeicherten Sollwerten auf. Bei einer unzulässigen Abweichung von den Sollwerten kann dann ein akustischer oder optischer Alarm (Blinken) ausgelöst werden.

In der Praxis wird die Diagnoseschaltung zweckmäßig so ausgeführt, daß sämtliche beschriebenen elektronischen Testfunktionen in einem mit der Auswerteelektronik in Verbindung stehenden Rechner als Software implementiert sind.

Mit der Erfindung werden folgende Vorteile erzielt:
- Das Gasspurenmeßsystem mit integrierter Diagnoseschaltung erlaubt eine einfache elektrische Funktionsüberwachung, mit der die Betriebsbereitschaft des Geräts jederzeit überprüft werden kann. Insbesondere werden keine zusätzlichen Gerätekomponenten, wie z.B. Kalibriereinheiten, benötigt. Durch die Diagnoseschaltung können die für die Erzeugung des Meßsignals verantwortlichen elektrochemischen Reaktionen in der Meßzelle kontrolliert werden. Zusätzlich wird die Funktion der analogen Meßwertverarbeitung in der Auswerteelektronik überwacht. Beim Auftreten eines Fehlersignals kann ein Alarm ausgelöst werden oder auf eine zweite, parallel betriebene Sensorzelle umgeschaltet werden.
- Da für den Testzyklus nur ein Bruchteil der Ansprechzeit der Meßzelle erforderlich ist (1- 20 s), wird der Meßvorgang, z.B. die kontinuierliche Überwachung einer Gaskonzentration praktisch nicht unterbrochen. Die Funktionskontrolle kann daher in periodischen Zeitabständen während des betrieblichen Einsatzes des Gasspurenmeßsystems erfolgen. Auf diese Weise können Fehlfunktionen rechtzeitig erkannt werden.
- Ein weiterer Vorteil der Erfindung liegt darin, daß die Diagnoseschaltung raumsparend mit geringem schaltungstechnischen Aufwand zu realisieren ist, so daß bereits bestehende Gerätegenerationen problemlos nachgerüstet werden können.

Im folgenden wird die Erfindung anhand eines in den Zeichnungen dargestellten Ausführungsbeispieles näher beschrieben. Es zeigen:
- Fig. 1: ein Blockschaltbild des Gasspurenmeßsystems einschließlich der Diagnoseschaltung,
- Fig. 2: zyklische Voltagramme bei einem Dreielektrodensensor,
- Fig. 3: ein differentielles zyklisches Voltagramm bei einem defekten Dreielektrodensensor, und
- Fig. 4: ein differentielles zyklisches Voltagramm bei einem intakten Dreielektrodensensor.

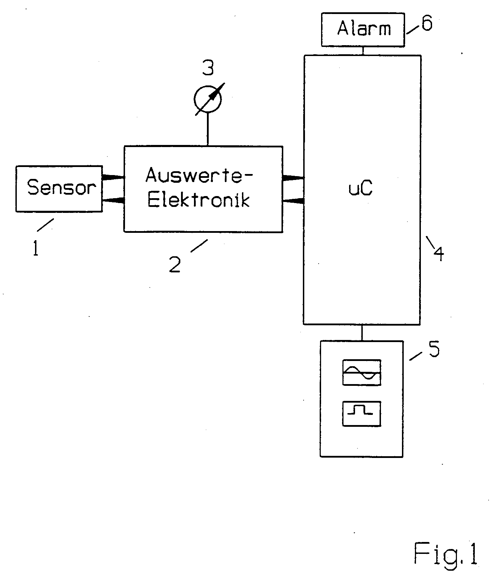

Das Gasspurenmeßsystem gemaß Fig. 1 besteht aus einem elektrochemischen Zwei- oder Dreielektrodensensor 1, einer Auswerteelektronik 2 mit einer Anzeige 3 und der Diagnoseschaltung 4, 5, 6. Die wesentlichen Komponenten der Diagnoseschaltung sind der Rechner 4, der Testspannungsgenerator 5 und der Alarmgeber 6. Der Sensor 1 erzeugt abhängig von der anstehenden Gaskonzentration ein Stromsignal, das von der analogen Auswerteelektronik verstärkt und vom Instrument 3 angezeigt wird. Außerdem sorgt die Auswerteelektronik 2 dafür, daß zwischen der Arbeitselektrode und der Gegenelektrode des Sensors 1 (bei einem Zweielektrodensensor) bzw. zwischen Arbeitselektrode und Referenzelektrode (bei einem Dreielektrodensensor) in Abhängigkeit von der zu messenden Gasart ein vorgegebenes Sollpotential U₀ eingestellt wird.

Wenn eine Funktionskontrolle des Sensors 1 durchgeführt werden soll, wird von der Diagnoseschaltung vom Meßmodus vorübergehend in einen Testmodus umgeschaltet. Im Testmodus wird dem Sollpotential U₀ an der Arbeitselektrode eine Testspannung ΔU überlagert. Die Testspannung ΔU kann entweder eine periodische Wechselspannung oder ein einmaliger Rechteckimpuls sein und wird von dem Testspannungsgenerator 5 geliefert. Die Umschaltung vom Meßmodus in den Testmodus erfolgt manuell, z.B. durch Betätigung eines Schalters, oder wird selbsttätig vom Rechner 4 in regelmäßigen Zeitabständen durchgeführt.

Die Änderung des Arbeitselektrodenpotentials hat eine Änderung des Stromsignals zur Folge, die für Testzwecke ausgenutzt wird. Diese Stromantwort des Sensors oder eine davon durch weitere Meßwertverarbeitung abgeleitete Größe (nachfolgend näher erläutert) wird hier als Testsignal bezeichnet. Die durch die Testspannung hervorgerufenen. Testsignale können z.B. direkt mit vorgegebenen Sollwerten verglichen werden, die für die Sensorqualität spezifisch sind und vorher bei fehlerfrei funktionierenden Sensorzellen empirisch bestimmt wurden. Diese Sollwerte sind ebenfalls im Rechner 4 gespeichert. Der Rechner 4 vergleicht auch die Testsignale mit den vorgegebenen Sollwerten (Komparatorfunktion) und gibt bei einer unzulässigen Überschreitung einen Alarm aus (Alarmgeber 6). Ferner kann das Zeitverhalten des Sensors, insbesondere die Ansprechzeit, durch eine Registrierung der zeitlichen Änderung des Testsignals untersucht werden. Zu diesem Zweck wird dem Sollpotential an der Arbeitselektrode als Testspannung ein kurzer Rechteckimpuls überlagert und das dadurch hervorgerufene Testsignal als Funktion der Zeit registriert. Im folgenden werden Ausführungsbeispiele für einen Funktionstest bei einem Dreielektroden- und Zweielektrodensensor beschrieben.

### 1. Dreielektrodensensor

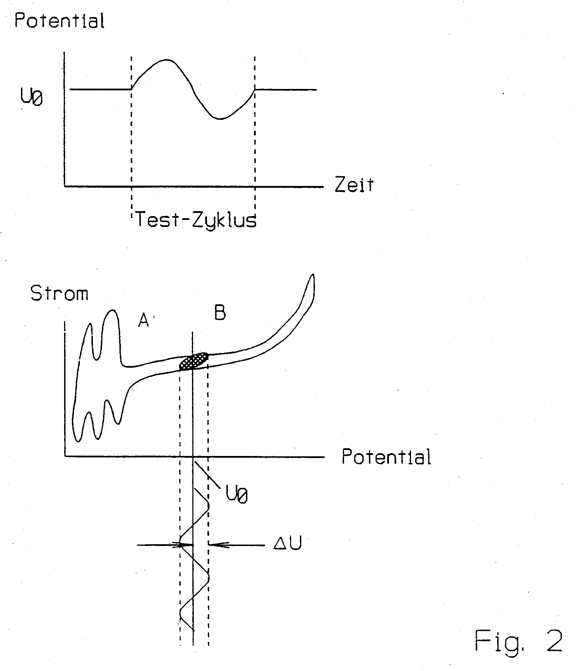

Mit einem potentiostatisch betriebenen Dreielektrodensensor wird für einen kurzen Testzyklus (typisch 1 bis 20 s) eine zyklische Voltametrie durchgeführt. Zu diesem Zweck wird das vorgegebene Sollpotential U₀ mit einer Sinuswechselspannung moduliert und das resultierende Stromsignal an der Arbeitselektrode gemessen. Die Amplitude ΔU der sinusförmigen Wechselspannung liegt im Bereich von 1 bis 200 mV, die Frequenz zwischen 0,01-10 s⁻¹, vorzugsweise 0,1-1 s⁻¹. In dem unteren Bild in Fig. 2 ist ein zyklisches Voltagramm (Kurve A) dargestellt, das man erhält, wenn das Arbeitselektrodenpotential (bezogen auf die Referenzelektrode) in einem weiten Bereich variiert wird und das zugehörige Stromsignal registriert wird. Ein solches zyklisches Voltagramm wird standardmäßig zur elektrochemischen Analyse von Katalysatoroberflächen und Elektrolyten herangezogen. Ein differentielles zyklisches Voltagramm (Kurve B) erhält man dagegen, wenn dem stationären Sollpotential U₀, wie oben beschrieben, eine Wechselspannung ΔU überlagert wird und die dazugehörende Stromsignaländerung an der Arbeitselektrode registriert wird. Von dem stationären Sollpotential U₀ ausgehend, wird so ein kleiner Ausschnitt des zyklischen Voltagramms mit der sich ergebenden Hysterese zwischen anodischem und kathodischem Durchlauf durchfahren (Kurve B). Die Hysterese entsteht durch den Aufbau bzw. den Abbau der Elektrodendeckschicht und ist von der Änderungsgeschwindigkeit des Potentials abhängig. Die Fläche dieser Hysteresekurve des differentiellen zyklischen Voltagramms, d.h. das Integral über die Differenz zwischen kathodischem und anodischem Durchlauf, liefert ein Kriterium für die Funktion des gesamten elektrochemischen Dreielektrodensensors. Aus diesem Grund wird hier die Hysteresefläche als Testsignal verwendet. Das Testsignal wird also hier im Rechner 4 durch Integration des in die Arbeitselektrode fließenden Stromsignals über eine Periode der Wechselspannung gebildet und mit vorgewählten Sollwerten verglichen.

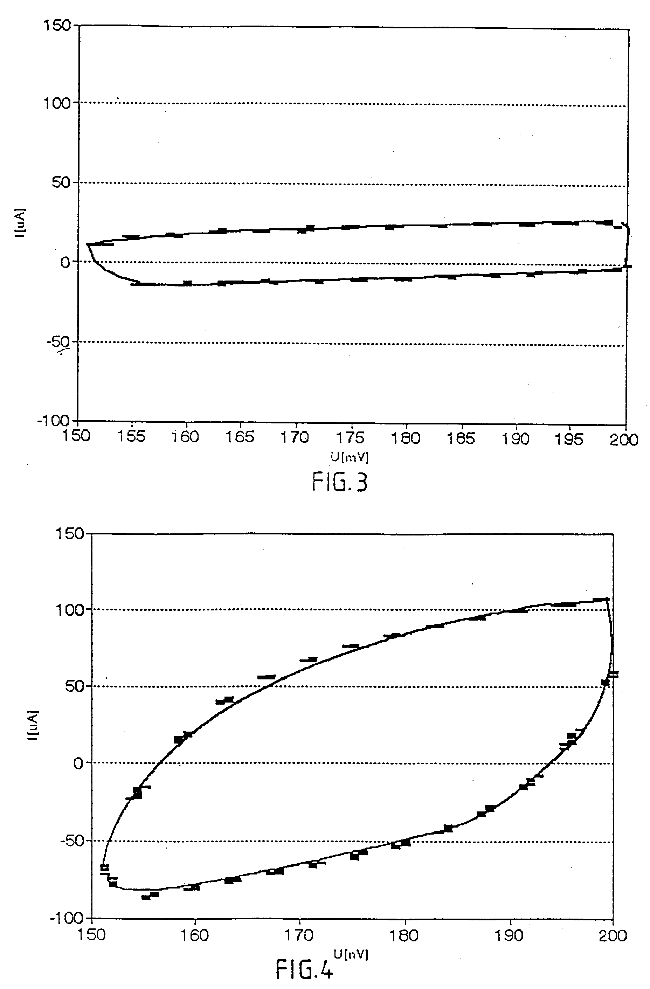

Fig. 3 zeigt ein experimentell registriertes, differentielles zyklisches Voltagramm einer defekten und Fig. 4 einer intakten Sensorzelle, die mit dem hier beschriebenen Testverfahren verglichen wurden. Die aufgenommenen Hysteresekurven bei einer Modulation der Arbeitspotentiale von 150 mV mit einer Wechselspannung von 50 mV zeigen einen deutlichen Unterschied zwischen den beiden Sensoren. Die ca. um den Faktor 5 unterschiedlichen Hystereseflächen ermöglichen eine klare Erkennung der defekten Zelle. Die Form und die Fläche der Hysteresekurve sind unabhängig von der Begasung der Zelle, so daß der Funktionstest auch direkt während einer Messung bei Begasung durchgeführt werden kann.

Ein Bruch einer der Elektrodenableitungen, Eintrocknen oder Auslaufen des Elektrolyten, fehlende Benetzung einer der Elektroden, Vergiftung der Katalysatoren und andere Fehler machen sich durch eine signifikante Veränderung der Hysteresefläche bemerkbar und können daher als Fehlfunktion erkannt werden und in ein Alarmsignal oder eine Fehlermeldung umgesetzt werden. Durch den kurzen Testzyklus wird praktisch die kontinuierliche Überwachung einer Gaskonzentration nicht gestört. Im übrigen kann der Testzyklus zusätzlich durch eine Speicherung des letzten Meßwertes überbrückt werden. In extrem zeitkritischen Fällen können zwei identische Zellen parallel betrieben werden, wobei der Funktionstest im Wechseltakt erfolgt und jeweils ein Sensor getestet wird, während der andere in den Meßmodus geschaltet ist und den kontinuierlichen Meßbetrieb sicherstellt.

### 2. Zweielektrodensensor

Das Sensormeßsignal ist in diesem Fall der bei einem voreingestellten Arbeitselektrodenpotential (U₀) amperometrisch gemessene Strom. Diesem Sollpotential wird mit Hilfe des Testspannungsgenerators 5 kurzzeitig ein Rechteckimpuls mit einer Dauer von 1 ms bis 100 ms und einer Amplitude von 1 bis 200 mV überlagert. Als Testspannung wird also hier keine periodische Wechselspannung, sondern ein einmaliger Spannungsimpuls verwendet. Dieser Spannungsimpuls hat eine charakteristische Stromantwort zur Folge, die hier als Testsignal benutzt wird und eine Information über die Funktionstüchtigkeit des Sensors liefert. Auch bei diesem Test werden Defekte der Sensorzelle als signifikante Änderung der Stromantwort erkannt.

Die beschriebenen Testfunktionen, wie Umschaltung in den Testmodus, Zuschaltung des Testspannungsgenerators 5, Integration und Differentiation der Signalspannungen, Vergleich mit Standardwerten, Alarmauslösung, können natürlich auch mit diskreten elektrischen Bauelementen oder Baugruppen realisiert werden. Die Diagnoseschaltung kann aber dann nicht so raumsparend aufgebaut werden wie bei Verwendung eines Mikroprozessors.

## Patentansprüche

1. Gasspurenmeßsystem bestehend aus einem elektrochemischen Sensor (1) mit mindestens einer Arbeitselektrode, einer Gegenelektrode und gegebenenfalls einer Referenzelektrode sowie einer analogen Auswerteelektronik (2) zur Bildung von Meßwerten für die zu untersuchenden Gaskonzentrationen, dadurch gekennzeichnet, daß die Auswerteelektronik (2) mit einer Diagnoseschaltung (4, 5, 6) verbunden ist, die während eines Testzyklus das zwischen Arbeitselektrode und Gegenelektrode oder zwischen Arbeitselektrode und Referenzelektrode eingestellte Potential nach einer vorgegebenen Zeitfunktion vorübergehend verändert und die dadurch erzeugte Stromsignaländerung an der Arbeitselektrode oder ein davon abgeleiteter Meßwert als ein für die Sensorqualität repräsentatives Testsignal ausgibt und mit einem oder mehreren vorgegebenen Sollwerten vergleicht.

2. Gasspurenmeßsystem nach Anspruch 1, dadurch gekennzeichnet, daß zur Untersuchung der Ansprechzeit des Sensors (1) die zeitliche Änderung des Testsignals gemessen wird.

3. Gasspurenmeßsystem nach Anspruch 1, dadurch gekennzeichnet, daß das Testsignal durch Integration des während des Testzyklus in die Arbeitselektrode fließenden Stromsignals erzeugt wird.

4. Gasspsurenmeßsystem nach Anspruch 3, dadurch gekennzeichnet, daß bei einem elektrochemischen Drei- oder Mehrelektrodensensor mit einer analogen potentiostatischen Auswerteelektronik das Potential zwischen Arbeitselektrode und Referenzelektrode während des Testzyklus nach Art eines differentiellen, zyklischen Voltagramms mit einer Wechselspannung moduliert wird und durch Integration des in die Arbeitselektrode fließenden Stromsignals über eine Periode der Wechselspannung die von der dabei durchlaufenen Stromspannungshysteresekurve eingeschlossene Fläche als Testsignal ausgegeben wird.

5. Gasspurenmeßsystem nach Ansprüchen 1 bis 4, dadurch gekennzeichnet, daß die Diagnoseschaltung einem Komparator zum Vergleich des Testsignals mit einem vorgegebenen, gespeicherten Sollwert und einen Alarmgeber (6) aufweist, der einen akustischen oder optischen Alarm ausgibt, wenn das Testsignal vom Sollwert abweicht.

6. Gasspurenmeßsystem nach Ansprüchen 1 bis 5, dadurch gekennzeichnet, daß die Diagnoseschaltung im wesentlichen aus einem Rechner (4) besteht, in dem sämtliche elektronische Testfunktionen als Software implementiert sind.
